# EUROPEAN PATENT SPECIFICATION

(11) **EP 2 477 975 B1**
(45) Date of publication and mention of the grant of the patent: **10.01.2018**
(21) Application number: 10817525.8
(22) Date of filing: 17.09.2010
(51) Int. Cl.: C07D 401/04, A61P 29/00, A61P 37/00, A61K 31/454, A61P 35/00

(54) **SOLID FORMS OF 3-(4-AMINO-1-OXO-1,3-DIHYDRO-ISOINDOL-2-YL)-PIPERIDINE-2,6-DIONE AND METHODS OF MAKING THE SAME**
FESTE FORMEN VON 3-(4-AMINO-1-OXO-1,3-DIHYDRO-ISOINDOL-2-YL)-PIPERIDIN-2,6-DION UND HERSTELLUNGSVERFAHREN DAFÜR
FORMES SOLIDES DE 3-(4-AMINO-1-OXO-1,3-DIHYDRO-ISOINDOL-2-YL)- PIPÉRIDINE-2,6-DIONE ET LEURS PROCÉDÉS DE PRÉPARATION

(30) Priority: 17.09.2009 US 243204 P
(43) Date of publication of application: 25.07.2012
(73) Proprietor: ScinoPharm Taiwan Ltd., Tainan County 741 (TW)
(72) Inventor: CHEN, Chen-Tung, Taiwan 74144 (TW); LIN, Inze, Taiwan 74144 (TW)
(74) Representative: Gulde & Partner
(86) International application number: PCT/SG2010/000345
(87) International publication number: WO 2011/034504

(56) References cited:
- WO-A1-2006/028964
- WO-A1-2010/054833
- WO-A1-2010/061209
- WO-A1-2011/033468
- WO-A1-2011/061611
- WO-A1-2011/111053
- WO-A2-2005/023192
- WO-A2-2009/114601
- WO-A2-2010/100476
- WO-A2-2010/129636
- US-A1- 2006 160 854
- US-A1- 2011 060 010
- DATABASE WPI Week 201103 Thomson Scientific, London, GB; AN 2010-M02867 XP002691114, -& CN 101 817 813 A (NANJING CAVENDISH BIOENGINEERING TECHNOLOGY CO LTD) 1 September 2010 (2010-09-01)
- CAIRA M R: "CRYSTALLINE POLYMORPHISM OF ORGANIC COMPOUNDS", TOPICS IN CURRENT CHEMISTRY, SPRINGER, BERLIN, DE, vol. 198, 1 January 1998 (1998-01-01), pages 163-208, XP001156954, ISSN: 0340-1022, DOI: 10.1007/3-540-69178-2_5 ISBN: 978-3-540-36760-4
- DAVID ALBERS ET AL: "Characterization of the Polymorphic Behaviour of an Organic Compound Using a Dynamic Thermal and X-ray Powder Diffraction Technique", ORGANIC PROCESS RESEARCH & DEVELOPMENT, vol. 11, 2007, pages 846-860,

## Description

### Field of Invention

The present invention is concerned with novel crystalline form of 3-(4-amino-1-oxo-1,3-dihydro-isoindol-2-yl)-piperidine-2,6-dione and process for their preparation, pharmaceutical compositions containing the novel form.

### Background

3-(4-amino-1-oxo-1,3-dihydro-Isoindol-2-yl)-piperidine-2,6-dione or lenalidomide is known to be useful in treating and preventing various diseases, including, but not limited to, inflammatory diseases, autoimmune diseases, and cancer. Form A is an anhydrous form disclosed in U.S. Patent 7,465,800.

### Summary

We have obtained a crystalline form of 3-(4-amino-1-oxo-1,3-dihydro-Isoindol-2-yl)-piperidine-2,6-dione that exhibit unique properties.

### Synthesis of Crystalline Form I

### Form I

Form I can be generally obtained by evaporation in a methanol/water solvent system. Form I can also be obtained from Form A in a hot water slurry system.
FIG. 1a-b is a characteristic X-ray powder diffraction pattern of Form I.
FIG. 2 is the Differential Scanning Calorimetry Pattern of Form I
FIG. 3 is the Thermogravimetric Analysis Pattern of Form I
FIG. 4 a-b is an infrared diffuse-reflectance pattern of Form I
FIG. 5 is a characteristic X-ray powder diffraction pattern of the amorphous form.
FIG. 6a-c is an infrared diffuse-reflectance pattern of amorphous form.

### Detailed Description

A crystalline form of 3-(4-amino-1-oxo-1,3-dihydro-isoindol-2-yl)-piperidine-2,6-dione having an X-ray powder diffraction pattern at 12.1±0.2, 13.4±0.2, 18.9±0.2, 23.9±0.2 and 25.8±0.2 degrees in two theta.

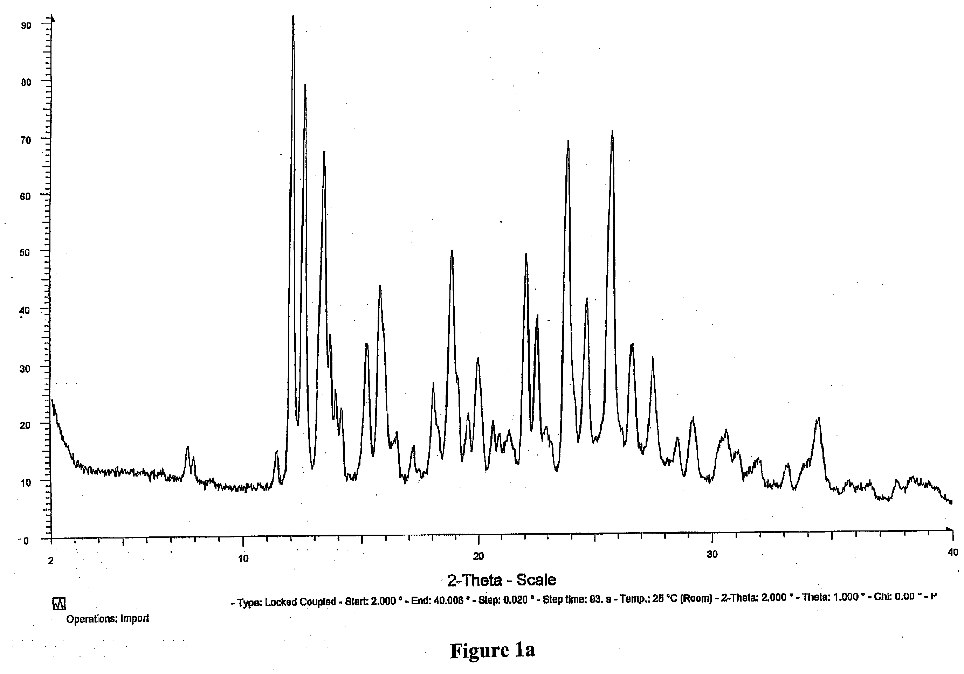

The crystalline form has X-ray powder diffraction pattern is in accordance with that shown in FIG. 1a-b.

The crystalline form may have an infrared diffuse-reflectance pattern with peaks at 1698, 1662, 1493, 1350 and 1203 wave numbers.

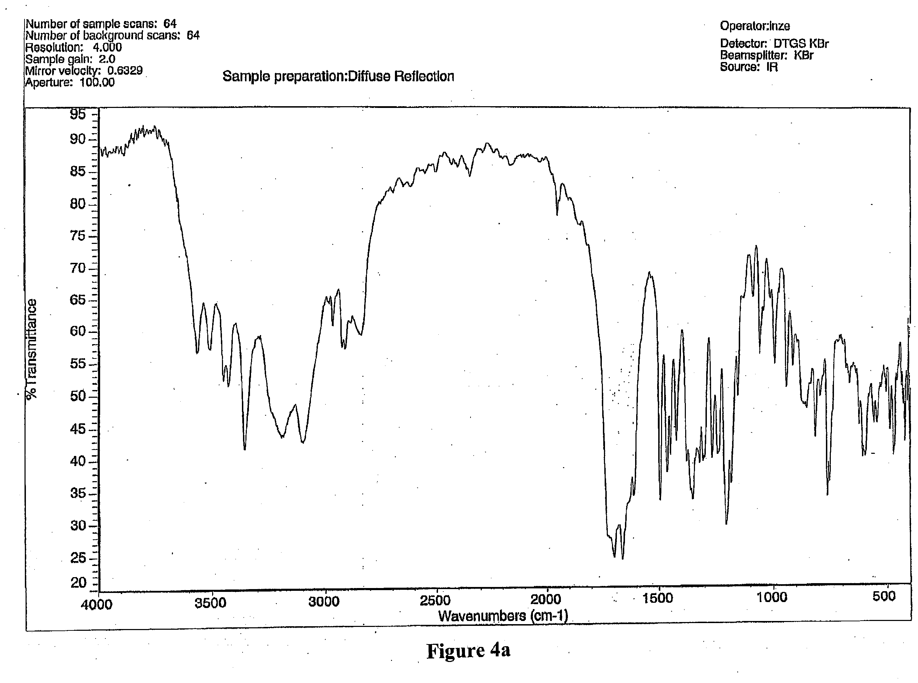

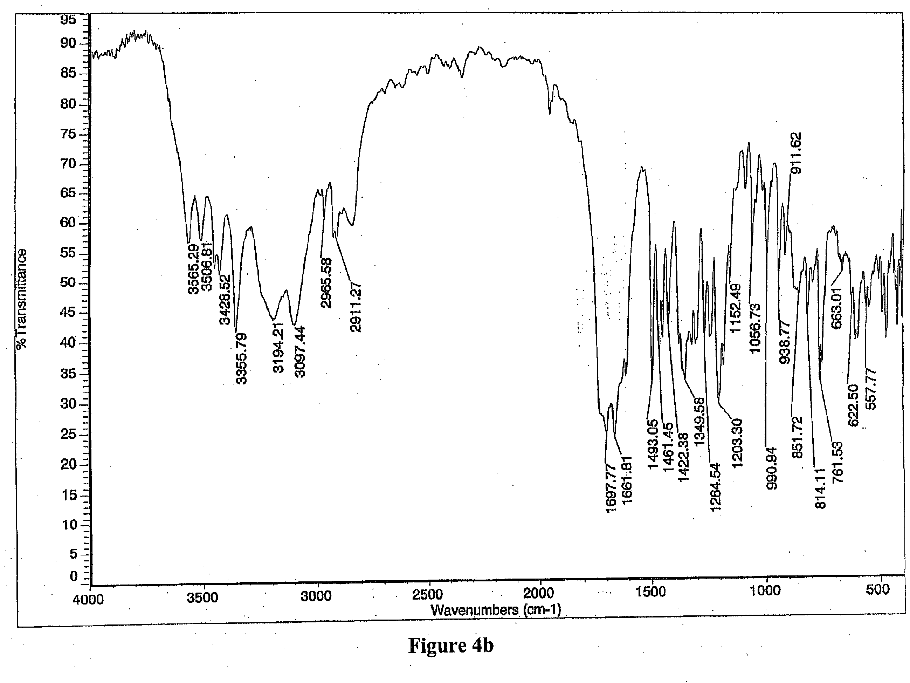

The crystalline form may have an infrared diffuse-reflectance pattern is in accordance with that shown in FIG. 4a-b.

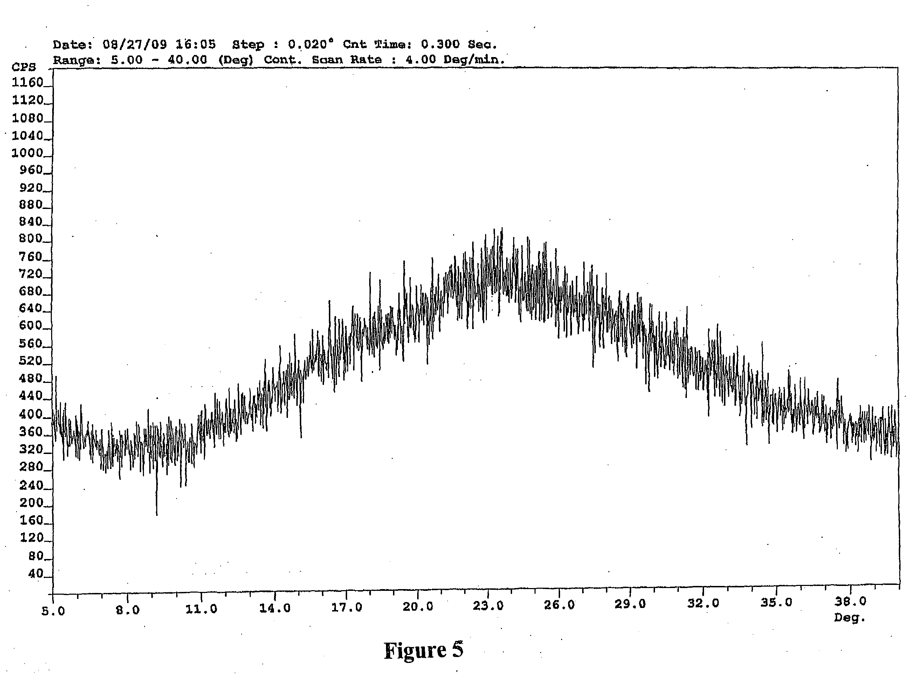

A process for preparing 3-(4-amino-1-oxo-1,3-dihydro-isoindol-2-yl)-piperidine-2,6-dione of the invention comprising:
a) heating a of 3-(4-amino-1-oxo-1,3-dihydro-isoindol-2-yl)-piperidine-2,6-dione in aqueous solution at a concentration of 10mg/ml for 3 hours at 60°C;
b) isolating the crystalline form of 3-(4-amino-1-oxo-1,3-dihydro-isoindol-2-yl)-piperidine-2,6-dione from the aqueous solution by filtration, and
c) drying the isolated crystalline form of 3-(4-amino-1-oxo-1,3-dihydro-isoindol-2-yl)-piperidine-2 6-dione for 28 hours at 40°C under reduced pressure. A process for preparing 3-(4-amino-1-oxo-1,3-dihydro-isoindol-2-yl)-piperidine-2,6-dione of the invention comprising:
   a) dissolving a 3-(4-amino-1-oxo-1,3-dihydro-isoindol-2-yl)-piperidine-2,6-dione in methanol to produce a first solution,
   b) removing a portion of the methanol from the first solution;
   c) adding water to produce a second mixture;
   d) removing a portion of the water and methanol from the second mixture, then isolate the crystalline form of 3-(4-amino-1-oxo-1,3-dihydro-isoindol-2-yl)-piperidine-2,6-dione, and
   e) drying the isolated crystalline form of 3-(4-amino-1-oxo-1,3-dihydro-isoindol-2-yl)-piperidine-2,6-dione. Described herein is an amorphous form of 3-(4-amino-1-oxo-1,3-dihydro-isoindol-2-yl)-piperidine-2,6-dione. The amorphous form may have the X-ray powder diffraction pattern is substantially in accordance with that shown in FIG. 5.

The amorphous form may have an infrared diffuse-reflectance pattern with peaks at approximately 1708, 1609, 1493, 1348 and 1206 wave numbers.

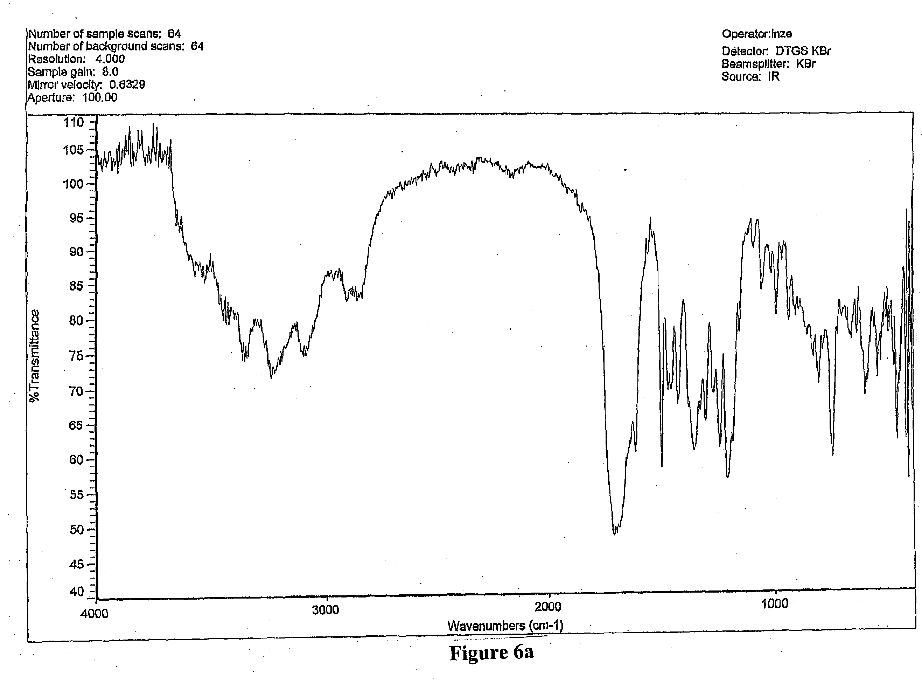

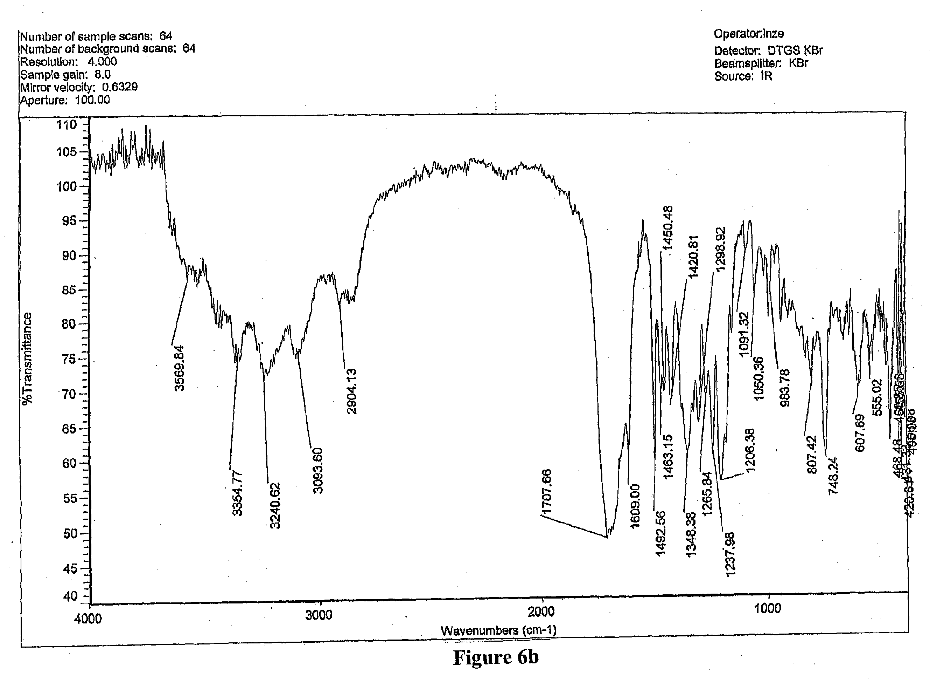

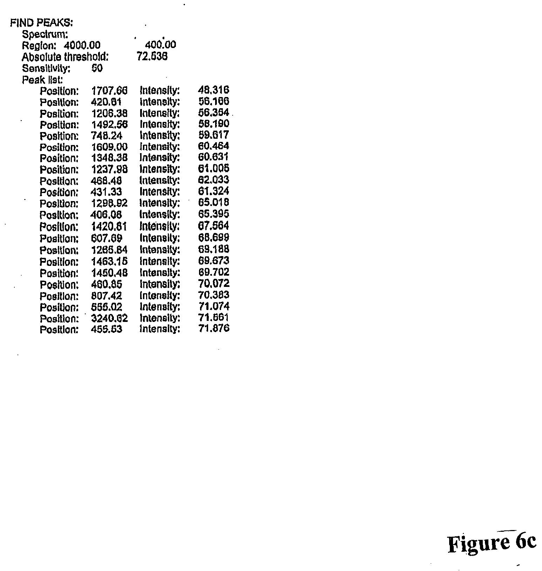

The amorphous form may have an infrared diffuse-reflectance pattern that is substantially in accordance with that shown in FIG. 6a-b. Described herein is a process for preparing amorphous form of 3-(4-amino-1-oxo-1,3-dihydro-isoindol-2-yl)-piperidine-2,6-dione of the invention comprising:
a) Dissolving 3-(4-amino-1-oxo-1,3-dihydro-isoindol-2-yl)-piperidine-2,6-dione in methanol, and
b) removing the solvent to obtain the amorphous form of 3-(4-amino-1-oxo-1,3-dihydro-isoindol-2-yl)-piperidine-2,6-dione.

A pharmaceutical composition comprising a therapeutically effective amount of the crystalline form I may include a pharmaceutically acceptable carrier. Described herein is a pharmaceutical composition comprising a therapeutically effective amount of the amorphous form which may include a pharmaceutically acceptable carrier.

### Preparation of 3-(4-amino-1-oxo-1,3-dihydro-isoindol-2-yl)-piperidine-2;6-dione

### Form I

Form I can be generally obtained by evaporation in a methanol/water solvent system. Form I can also be obtained from Form A in a hot water slurry system. Form A is an anhydrous form disclosed in U.S. Patent 7,465,800. Specific examples of both methods are shown below.

### Example 1:

A mixture of Form A 3-(4-amino-1-oxo-1,3-dihydro-Isoindol-2-yl)-piperidine-2,6-dione (300 mg) and aqueous solution (30 ml) was heated for 3 hours at 60°C. The mixture was filtered and the wet cake was dried for about 28 hours at 40°C under reduced pressure. The solid was analyzed by XRD and IR and identified as form I.

### Example 2

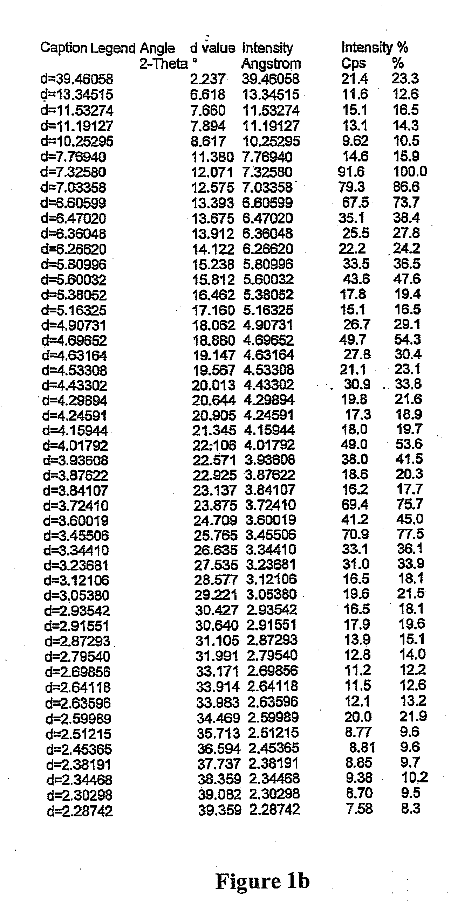

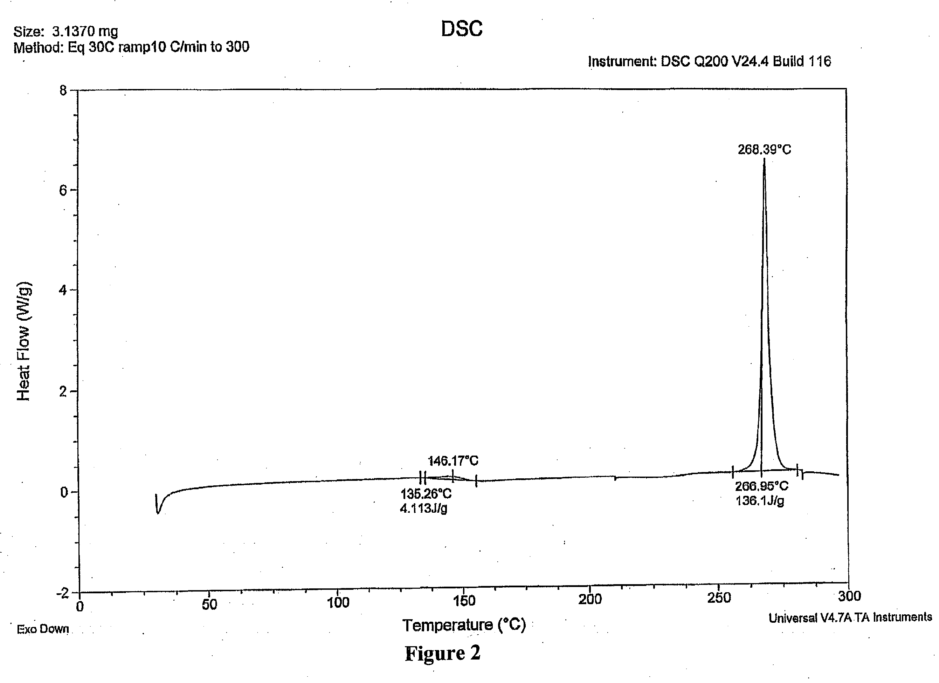

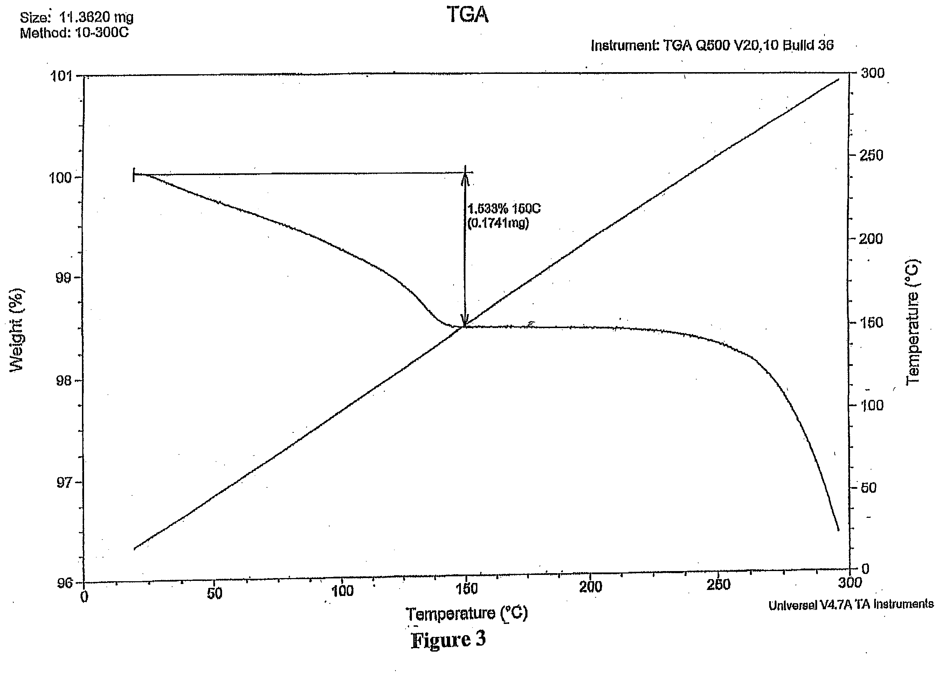

A 500 ml jacket reactor was charged with a solution of 3-(4-amino-1-oxo-1,3 dihydro-isoindol-2-yl)-piperidine-2,6-dione (about 1.3g) and methanol (about 260ml). The solution was heated to 60°C. The solution was concentrated to remove around 2/3 of methanol and the slurry solution was charged with water (about 300 ml). The mixture was further concentrated to remove methanol. The resulting slurry was filtered and the wet cake was dried at 40°C under reduced pressure for around 35 hours. The dried sample was submitted for XRPD, TGA, DSC and infrared diffuse reflectance analysis. The results of the analysis are shown in Figs. 1-4.

### Reference example: preparation of the amorphous form of 3-(4-amino-1-oxo-1,3-dihydro-isoindol-2-yl)-piperidine-2,6-dione

### Example 3:

Crystalline 3-(4-amino-1-oxo-1,3-dihydro-isoindol-2-yl)-piperidine-2,6-dione (about 300 mg) was dissolved in methanol (50 ml) at 60°C to obtain a clear solution. The solution was concentrated under reduced pressure to remove methanol and solid was obtained. The solid substance was analyzed by XRD and infrared diffuse reflectance analysis, and identified as an amorphous form, see Figures 5-6a-c.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

## Claims

1. A crystalline form of 3-(4-amino-1-oxo-1,3-dihydro-isoindol-2-yl)-piperidine-2,6-dione having an X-ray powder diffraction pattern in accordance with that shown in FIG. 1a-b; and having a TGA pattern as shown in Fig. 3.

2. The crystalline form of claim 1 wherein the X-ray powder diffraction pattern having peaks at 12.1±0.2, 12.6± 0.2, 13.4±0.2, 18.9±0.2, 20.0± 0.2, 23.9±0.2, 24.7±0.2, 25.8±0.2, 28.6±0.2 degrees in two theta.

3. The crystalline form of claim 1 having an infrared diffuse-reflectance pattern with peaks at 1698, 1662, 1493, 1350 and 1203 cm⁻¹ wavenumbers.

4. The crystalline form of claim 1 having an infrared diffuse-reflectance pattern is in accordance with that shown in FIG. 4a-b.

5. A process for preparing 3-(4-amino-1-oxo-1,3-dihydro-isoindol-2-yl)-piperidine-2,6-dione of claim 1 comprising:
(a) heating 3-(4-amino-1-oxo-1,3-dihydro-isoindol-2-yl)-piperidine-2,6-dione in aqueous solution at a concentration of 10mg/ml for 3 hours at 60°C,
(b) isolating the crystalline form of 3-(4-amino-1-oxo-1,3-dihydro-isoindol-2-yl)-piperidine-2,6-dione from the aqueous solution by filtration, and
(c) drying the isolated crystalline form of 3-(4-amino-1-oxo-1,3-dihydro-isoindol-2-yl)-piperidine-2,6-dione for 28 hours at 40°C under reduced pressure.

6. A process for preparing 3-(4-amino-1-oxo-1,3-dihydro-isoindol-2-yl)-piperidine-2,6-dione of claim 1 comprising:
(a) dissolving a 3-(4-amino-1-oxo-1,3-dihydro-isoindol-2-yl)-piperidine-2,6-dione in methanol to produce a first solution,
(b) removing a portion of the methanol from the first solution;
(c) adding water to produce a second mixture;
(d) removing a portion of the water and methanol from the second mixture, then isolate the crystalline form of 3-(4-amino-1-oxo-1,3-dihydro-isoindol-2-yl)-piperidine-2,6-dione, and
(e) drying the isolated crystalline form of 3-(4-amino-1-oxo-1,3-dihydro-isoindol-2-yl)-piperidine-2,6-dione.

7. A pharmaceutical composition comprising a therapeutically effective amount of the crystalline form I of claim 1 and a pharmaceutically acceptable carrier.

## Patentansprüche

1. Kristalline Form von 3-(4-Amino-1-oxo-1,3-dihydro-isoindol-2-yl)-piperidin-2,6-dion mit einem Röntgenpulverdiffraktogramm, das dem in Fig. 1a-b dargestellten entspricht; und mit einem TGA-Diagramm wie in Fig. 3 gezeigt.

2. Kristalline Form nach Anspruch 1, wobei das Röntgenpulverdiffraktogramm Peaks bei 12,1° ± 0,2°, 12,6° ± 0,2°, 13,4° ± 0,2°, 18,9° ± 0,2°, 20,0° ± 0,2°, 23,9° ± 0,2°, 24,7° ± 0,2°, 25,8° ± 0,2°, 28,6° ± 0,2° (20) aufweist.

3. Kristalline Form nach Anspruch 1 mit einem Infrarotspektrum der diffusen Reflexion mit Peaks bei den Wellenzahlen 1698, 1662, 1493, 1350 und 1203 cm⁻¹.

4. Kristalline Form nach Anspruch 1 mit einem Infrarotspektrum der diffusen Reflexion, das dem in Fig. 4a-b dargestellten entspricht.

5. Verfahren zur Herstellung von 3-(4-Amino-1-oxo-1,3-dihydro-isoindol-2-yl)-piperidin-2,6-dion nach Anspruch 1, umfassend:
(a) Erwärmen von 3-(4-Amino-1-oxo-1,3-dihydro-isoindol-2-yl)-piperidin-2,6-dion in wässriger Lösung in einer Konzentration von 10 mg/ml über 3 Stunden bei 60 °C,
(b) Abtrennen der kristallinen Form von 3-(4-Amino-1-oxo-1,3-dihydro-isoindol-2-yl)-piperidin-2,6-dion aus der wässrigen Lösung durch Filtration, und
(c) Trocknen der abgetrennten kristallinen Form von 3-(4-Amino-1-oxo-1,3-dihydro-isoindol-2-yl)-piperidin-2,6-dion über 28 Stunden bei 40 °C unter verringertem Druck.

6. Verfahren zur Herstellung von 3-(4-Amino-1-oxo-1,3-dihydro-isoindol-2-yl)-piperidin-2,6-dion nach Anspruch 1, umfassend:
(a) Lösen von 3-(4-Amino-1-oxo-1,3-dihydro-isoindol-2-yl)-piperidin-2,6-dion in Methanol, um eine erste Lösung herzustellen,
(b) Entfernen eines Teils des Methanols aus der ersten Lösung;
(c) Zugeben von Wasser, um ein zweites Gemisch herzustellen;
(d) Entfernen eines Teils des Wassers und des Methanols aus dem zweiten Gemisch, danach Abtrennen der kristallinen Form von 3-(4-Amino-1-oxo-1,3-dihydro-isoindol-2-yl)-piperidin-2,6-dion, und
(e) Trocknen der abgetrennten kristallinen Form von 3-(4-Amino-1-oxo-1,3-dihydro-isoindol-2-yl)-piperidin-2,6-dion.

7. Pharmazeutische Zusammensetzung, umfassend eine therapeutisch wirksame Menge der kristallinen Form I nach Anspruch 1 und einen pharmazeutisch akzeptablen Träger.

## Revendications

1. Forme cristalline de 3-(4-amino-1-oxo-1,3-dihydro-isoindol-2-yl)-pipéridine-2,6-dione ayant un diagramme de diffraction des rayons X sur poudre conforme à celui montré sur la Fig. 1a-b ; et ayant un diagramme ATG comme montré sur la Fig. 3.

2. Forme cristalline selon la revendication 1, le diagramme de diffraction des rayons X sur poudre ayant des pics à 12,1±0,2, 12,6±0,2, 13,4±0,2, 18,9±0,2, 20,0±0,2, 23,9±0,2, 24,7±0,2, 25,8±0,2, 28,6±0,2 degrés en 2 Theta.

3. Forme cristalline selon la revendication 1 ayant un diagramme de réflectance diffuse infrarouge avec des pics aux nombres d'ondes 1 698, 1 662, 1 493, 1 350 et 1 203 cm⁻¹.

4. Forme cristalline selon la revendication 1 ayant un diagramme de réflectance diffuse infrarouge conforme à celui montré sur la Fig. 4a-b.

5. Procédé de préparation de la 3-(4-amino-1-oxo-1,3-dihydro-isoindol-2-yl)-pipéridine-2,6-dione de la revendication 1, comprenant :
(a) chauffer de la 3-(4-amino-1-oxo-1,3-dihydro-isoindol-2-yl)-pipéridine-2,6-dione en solution aqueuse à une concentration de 10 mg/ml pendant 3 heures à 60 °C,
(b) isoler la forme cristalline de 3-(4-amino-1-oxo-1,3-dihydro-isoindol-2-yl)-pipéridine-2,6-dione de la solution aqueuse par filtration, et
(c) chauffer la forme cristalline isolée de 3-(4-amino-1-oxo-1,3-dihydro-isoindol-2-yl)-pipéridine-2,6-dione pendant 28 heures à 40 °C à pression réduite.

6. Procédé de préparation de la 3-(4-amino-1-oxo-1,3-dihydro-isoindol-2-yl)-pipéridine-2,6-dione de la revendication 1, comprenant :
(a) dissoudre une 3-(4-amino-1-oxo-1,3-dihydro-isoindol-2-yl)-pipéridine-2,6-dione dans du méthanol pour produire une première solution,
(b) ôter une portion du méthanol de la première solution ;
(c) ajouter de l'eau pour produire un deuxième mélange ;
(d) ôter une portion de l'eau et du méthanol du deuxième mélange, puis isoler la forme cristalline de 3-(4-amino-1-oxo-1,3-dihydro-isoindol-2-yl)-pipéridine-2,6-dione, et
(e) sécher la forme cristalline isolée de 3-(4-amino-1-oxo-1,3-dihydro-isoindol-2-yl).pipéridine-2,6-dione.

7. Composition pharmaceutique comprenant une quantité efficace d'un point de vue thérapeutique de la forme cristalline I selon la revendication 1 et un support acceptable d'un point de vue pharmaceutique.
